# EUROPEAN PATENT APPLICATION

(11) **EP 1 891 892 A1**
(43) Date of publication of application: **27.02.2008**
(21) Application number: 06757026.7
(22) Date of filing: 06.06.2006
(51) Int. Cl.: A61B 5/00

(54) **BIOMETRIC INFORMATION MEASURING SENSOR**

(30) Priority: 07.06.2005 JP 2005166887
(71) Applicant: Omron Healthcare Co., Ltd., Kyoto 615-0084 (JP)
(72) Inventor: TOKITA, Muneo c/o OMRON HEALTHCARE CO., LTD., Kyoto-shi, Kyoto 6150084 (JP)
(74) Representative: Wilhelms . Kilian & Partner Patentanwälte
(86) International application number: PCT/JP2006/311286
(87) International publication number: WO 2006/132219

(57) **Abstract**

A biological information measuring sensor (10) has a light-receiving region (17) provided at a light-receiving element (16), a waveguide (15) provided in a waveguide formation member (14), filters (18a, 18b) for separating light emitted from an outlet-side opening portion (14b) of the waveguide (15) and irradiating the light-receiving region (17) with only light of a specific wavelength region, and a solenoid (20) for switching the filters (18a, 18b). The solenoid (20) switches between the filters (18a, 18b) to realize a first state where the light-receiving region (17) is irradiated with only light of a first wavelength region and a second state where the light-receiving region (17) is irradiated with only light of a second wavelength region. The construction simultaneously achieves both a reduction in size of a probe section and improvement of measurement accuracy.

## Description

### TECHNICAL FIELD

The present invention relates to a biological information measuring sensor for measuring biological information in a noninvasive manner by receiving light from a living body.

### BACKGROUND ART

Measurement of biological information is frequently carried out in the medical field. To measure biological information is very important to know the health conditions of a subject. Here, biological information includes a concentration of a particular component included in a living tissue, temperature information, heart rate, blood pressure, and the like. Particular components included in a living tissue to be measured include, for example, glucose, hemoglobin, oxyhemoglobin, neutral fat, cholesterol, albumin, uric acid, and the like included in the blood.

For example, in an ear thermometer, temperature information is measured by receiving radiation light radiated from the eardrum positioned in the auditory meatus. Furthermore, in a glucometer, a technique has been developed to measure a blood glucose concentration spectroscopically in a noninvasive manner by receiving radiation light radiated from the eardrum positioned in the auditory meatus by separating light of a wavelength in a particular range, and then detecting the spectrum of the received light. In addition, in an apparatus for measuring oxygen saturation, a finger sphygmomanometer, and the like, the technique of measuring biological information optically or spectroscopically in a noninvasive manner by receiving light from a living body has been established.

As described above, in the noninvasive measurement method in which light from a living body is received for optical or spectroscopic measurement in a noninvasive manner, it is not necessary to take a living tissue as represented by blood or humor as a sample from a subject, so that the burden on the subject is greatly reduced and the method is suitable as a method of measuring biological information.

When biological information is measured in a noninvasive manner using the optical or spectroscopic measurement method as described above, a biological information measuring sensor is used which receives radiation light emitted from a living body or transmitted light transmitted through a living body or reflected light reflected on a living body by light-receiving means and photoelectrically converting the same for output. The documents disclosing such a biological information measuring sensor include, for example, Japanese Patent Laying-Open No. 2003-70751 (Patent Document 1), Japanese National Publication No. 2001-503999 (Patent Document 2), and the like.

Among these, the biological information measuring sensor disclosed in the aforementioned Patent Document 2 measures a blood glucose concentration, in which light with two or more wavelengths in the mid-infrared range radiated from the eardrum is captured and the blood glucose concentration is measured based on the output. Therefore, in the biological information measuring sensor disclosed in the aforementioned Patent Document 2, a waveguide is formed of a waveguide tube having a straight pipe shape with a relatively large diameter, and two light-receiving regions are arranged at the back thereof.
Patent Document 1: Japanese Patent Laying-Open No. 2003-70751
Patent Document 2: Japanese National Publication No. 2001-503999

### DISCLOSURE OF THE INVENTION

### PROBLEMS TO BE SOLVED BY THE INVENTION

In general, radiation light radiating from a living body is very weak. Therefore, in order to measure biological information with high accuracy, it is necessary to increase the amount of light received. In order to increase the amount of light received, it is effective to enlarge the area of the light-receiving region. However, when the configuration disclosed in the aforementioned Patent Document 2 is employed, because of the presence of two light-receiving regions, the outlet-side opening portion of the waveguide needs to be increased in size correspondingly in accordance with the larger light-receiving region, and there also arises the need for increasing the size of the waveguide formation member, accordingly. However, if the waveguide formation member is increased in size, the probe portion is also increased in size accordingly, thereby making it difficult to put the probe portion into a narrow space such as an auditory meatus and, conversely, making it difficult to receive radiation light from a living body.

The present invention is therefore made to solve the aforementioned problem. An object of the present invention is to provide a biological information measuring sensor which achieves both size reduction of a probe portion and high-accuracy measurement.

### MEANS FOR SOLVING THE PROBLEMS

A biological information measuring sensor based on the present invention for measuring biological information in a noninvasive manner by receiving light from a living body includes a light-receiving region, a waveguide, spectroscopic means and switching means. The light-receiving region is provided in light-receiving means for receiving light from a living body. The waveguide includes an inlet-side opening portion where the light enters and an outlet-side opening portion where the light exits and is provided corresponding to the aforementioned light-receiving region to introduce the light to the aforementioned light-receiving region. The spectroscopic means is positioned between the aforementioned light-receiving region and the aforementioned waveguide to separate the light exiting from the outlet-side opening portion and apply only light having a wavelength in a particular range to the aforementioned light-receiving region. The switching means switches the aforementioned spectroscopic means to at least two or more states including a first state in which only light having a wavelength in a first range of the light is applied to the aforementioned light-receiving region and a second state in which only light having a wavelength in a second range of the light is applied to the aforementioned light-receiving region.

Here, "light from a living body" as mentioned above includes radiation light emitted from a living body as well as transmitted light and reflected light of light applied from a light source to a living body.

In this manner, because of the configuration in which the spectroscopic means can be switched by the switching means to at least two or more states including the first state and the second state, light with wavelengths in a plurality of ranges can be received separately at a single light-receiving region, which eliminates the need for providing a plurality of light-receiving regions. Therefore, even when the light-receiving region is increased in size, the opening diameter of the waveguide can be reduced as compared with the case where there are a plurality of light-receiving regions, thereby enabling size reduction of the probe portion of the apparatus including the biological information measuring sensor. Therefore, both improvement of measurement accuracy resulting from enlargement of the light-receiving region and size reduction of the probe portion can be achieved, resulting in a high-performance biological information measuring sensor. In addition, because of size reduction of the probe portion, when the probe portion is put into the auditory meatus or the like, the tip end of the probe portion can be brought closer to a part to be detected without coming into contact with the surrounding obstruction, so that the inlet-side opening portion of the waveguide can be brought into close proximity to a part to be detected. Therefore, more light can be introduced to the waveguide, and also in this respect, improvement of measurement accuracy can be achieved.

In the biological information measuring sensor based on the present invention as described above, the aforementioned spectroscopic means is preferably formed of a plurality of filters at least including a first filter only transmitting light having a wavelength in the first range and a second filter only transmitting light having a wavelength in the second range. In this case, preferably, the aforementioned switching means switches the plurality of filters such that only a particular filter of the plurality of filters is positioned between the aforementioned waveguide and the aforementioned light-receiving means.

In this manner, when a filter is used as spectroscopic means, a plurality of filters are configured to be switchable, so that light having wavelengths in a plurality of ranges can be received separately at a single light-receiving region.

In the biological information measuring sensor based on the present invention as described above, the aforementioned plurality of filters are preferably fixed to a filter fixing frame, and in this case, the aforementioned switching means is preferably formed of drive means for driving the aforementioned filter fixing frame.

In this manner, a plurality of filters are fixed to the filter fixing frame and the filter fixing frame is driven using the drive means, thereby realizing switching of the filters with a simple configuration. In this case, the drive means can be provided not in the probe portion of the apparatus but in the main body unit, thereby avoiding size increase of the probe portion by separate provision of drive means.

In the biological information measuring sensor based on the present invention as described above, the aforementioned filter fixing frame preferably has a shield region which does not transmit light. In this case, the aforementioned drive means preferably drives the aforementioned filter fixing frame, in addition to the first and second states, to a third state in which the light is not applied to the aforementioned light-receiving region by positioning the aforementioned shield region between the waveguide and the light-receiving region.

Because of such a configuration, monitoring of a room temperature state, detection of a reference output value, and the like become possible in the third state, so that such information can be used as parameters in measurement of biological information, resulting in a small and high-performance biological information measuring sensor.

In the biological information measuring sensor based on the present invention as described above, the aforementioned waveguide is preferably formed to have its opening area gradually decreasing from the inlet-side opening portion toward the outlet-side opening portion.

Because of such a configuration, the light entering the waveguide can be condensed in the outlet-side opening portion with the required minimum number of times of reflection on the wall surface of the waveguide. Therefore, absorption or scattering of light involved with reflection can be reduced as a whole, thereby realizing higher light-receiving efficiency.

In the biological information measuring sensor based on the present invention as described above, the aforementioned waveguide is preferably formed such that an opening shape in the inlet-side opening portion and an opening shape in the outlet-side opening portion are different from each other.

Because of such a configuration, at the inlet-side of the waveguide, the shape of the inlet-side opening portion can be selected so that light enters the waveguide to a maximum extent, while at the outlet-side of the waveguide, the shape of the outlet-side opening portion can be selected according to the shape of the light-receiving region so that the condensed light enters the light-receiving region without loss. Therefore, the higher light-receiving efficiency can be realized.

### EFFECTS OF THE INVENTION

In accordance with the present invention, a biological information measuring sensor is provided which achieves both size reduction of a probe portion and high-accuracy measurement.

### BRIEF DESCRIPTION OF THE DRAWINGS

Fig. 1 is a view schematically showing a usage state of a biological information measuring sensor in a first embodiment of the present invention
Fig. 2 is a schematic cross-sectional view of the biological information measuring sensor shown in Fig. 1.
Fig. 3 is a view showing a state after switching in a case where filters are switched in the biological information measuring sensor shown in Fig. 2.
Fig. 4A is a view showing a layout of filters of the biological information measuring sensor shown in Fig. 1 to Fig. 3.
Fig. 4B is a view showing another exemplary layout of filters of the biological information measuring sensor shown in Fig. 1 to Fig. 3.
Fig. 4C is a view showing yet another exemplary layout of filters of the biological information measuring sensor shown in Fig. 1 to Fig. 3.
Fig. 5 is a view schematically showing a usage state of a biological information measuring sensor in a second embodiment of the present invention.
Fig. 6 is a schematic cross-sectional view of the biological information measuring sensor shown in Fig. 5.
Fig. 7A is a view showing a layout of filters of the biological information measuring sensor shown in Fig. 5 and Fig. 6.
Fig. 7B is a view showing another exemplary layout of filters of the biological information measuring sensor shown in Fig. 5 and Fig. 6.
Fig. 7C is a view showing yet another exemplary layout of filters of the biological information measuring sensor shown in Fig. 5 and Fig. 6.
Fig. 8A is a view showing yet another exemplary layout of filters of the biological information measuring sensor shown in Fig. 5 and Fig. 6.
Fig. 8B is a view showing yet another exemplary layout of filters of the biological information measuring sensor shown in Fig. 5 and Fig. 6.
Fig. 9 is a view showing a modification of a waveguide formation member of the biological information measuring sensor in the first or second embodiment, in which (a) is a side view of the waveguide formation member, (b) is a view showing the shape of an inlet-side end face of the waveguide formation member, and (c) is a view showing the shape of an outlet-side end face of the waveguide formation member.

### DESCRIPTION OF THE REFERENCE SIGNS

10 biological information measuring sensor, 11 probe portion, 12 protective casing, 13 dustproof film, 13a dustproof window, 14 waveguide formation member, 14a inlet-side opening portion, 14b outlet-side opening portion, 15 waveguide, 16 light-receiving element, 17 light-receiving region, 18a first filter, 18b second filter, 18c third filter, 19 filter fixing frame, 19a shield region, 20 solenoid, 21 motor, 22 control portion, 23 light receiving circuit, 24 drive circuit, 30 eardrum (part to be detected).

### BEST MODES FOR CARRYING OUT THE INVENTION

In the following, embodiments of the present invention will be described in detail with reference to the figures. It is noted that in the embodiments illustrated below, a description will be made by way of illustration to a case where the present invention is applied to a glucometer for measuring a blood glucose concentration by putting a probe portion containing a biological information measuring sensor into the auditory meatus, and detecting the spectra of wavelengths in two ranges of mid-infrared radiation radiated from the eardrum using the biological information measuring sensor.

### (First Embodiment)

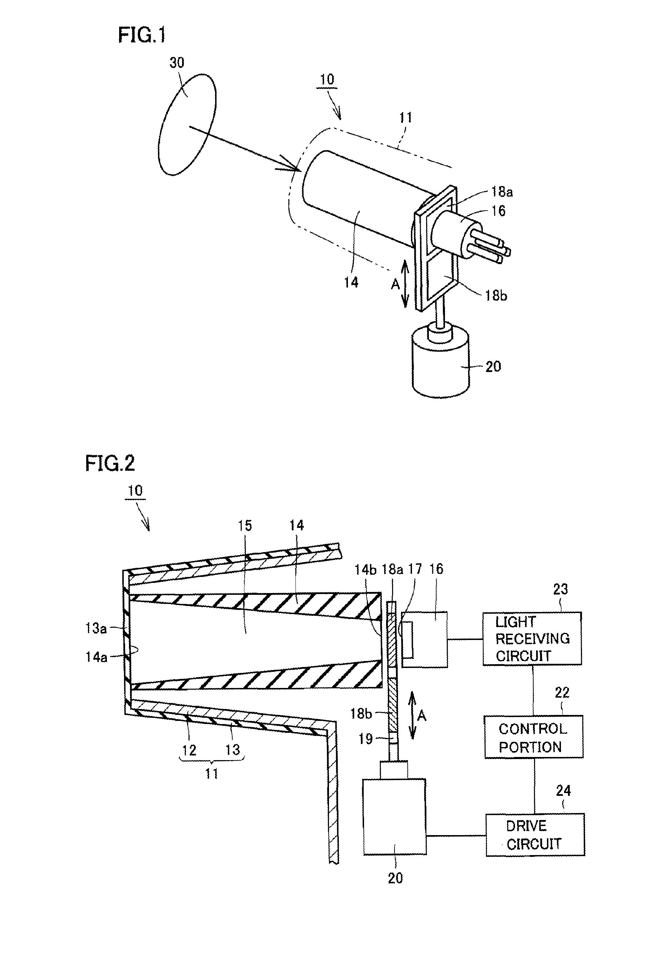

Fig. 1 is a view schematically showing a usage state of a biological information measuring sensor in a first embodiment of the present invention, and Fig. 2 is a schematic cross-sectional view of the biological information measuring sensor shown in Fig. 1. In the following, referring to these figures, the structure of the biological information measuring sensor in the present embodiment will be described.

As shown in Fig. 1, a biological information measuring sensor 10 in the present embodiment is included in a detection end of a glucometer to detect weak radiation light radiated from an eardrum 30 as a part to be detected, which is positioned in the auditory meatus. In measurement, as shown in Fig. 1, a probe portion 11 which is the detection end is inserted in the auditory meatus, and the front face (tip end face) of the inserted probe portion 11 is opposed to eardrum 30. In this state, radiation light radiated from eardrum 30 is introduced from the front face of probe portion 11 into probe portion 11, whereby measurement of a blood glucose concentration is performed.

As shown in Fig. 1 and Fig. 2, biological information measuring sensor 10 is mainly configured with a tubular waveguide formation member 14 arranged in the interior of probe portion 11 formed of a protective casing 12 and a dustproof film 13, and a light-receiving element 16 as light-receiving means, first and second filters 18a, 18b as spectroscopic means and a solenoid 20 as switching means and drive means, which are arranged in the interior of the apparatus body.

The above-noted protective casing 12 is formed of a tubular member having a front opening. Dustproof film 13 is attached to protective casing 12 to close the front opening of protective casing 12, and in particular, the part closing the front opening of protective casing 12 functions as a dustproof window 13a. Dustproof film 13 is a film for preventing intrusion of dust into the interior of probe portion 11, and a thin film of, for example, plastic, glass, silicon, or germanium is used for this dustproof film 13. In the present embodiment, a polyethylene film is used so that radiation light radiated from eardrum 30 is transmitted well.

As shown in Fig. 2, waveguide formation member 14 has a waveguide 15 in the interior thereof. Waveguide formation member 14 is formed, for example, of a resin material, a metal material or the like, and the inner circumferential surface defining waveguide 15 formed in the interior thereof is mirror-finished. A variety of methods can be adopted as the method of mirror finish, and, for example, gold plating and deposition of gold, aluminum or the like are suitable.

Waveguide formation member 14 is arranged such that the front face thereof faces dustproof window 13a of probe portion 11. At the back of waveguide formation member 14, the aforementioned light-receiving element 16 is arranged. Waveguide formation member 14 is provided with an inlet-side opening portion 14a on the front face where radiation light radiated from eardrum 30 enters and an outlet-side opening portion 14b on the back face where the aforementioned radiation light passing through waveguide 15 exits. Waveguide 15 is formed such that its opening area gradually decreases from inlet-side opening portion 14a toward outlet-side opening portion 14b.

Light-receiving element 16 arranged at the back of waveguide formation member 14 is an element photoelectrically converting an optical signal received at the light-receiving region as described later into an electrical signal. On the main surface of light-receiving element 16, a light-receiving region 17 is provided. This light-receiving region 17 is a region receiving light from a living body. As light-receiving element 16, for example, a photodiode may be used.

At the back of waveguide formation member 14 and in front of light-receiving region 17, a filter fixing frame 19 to which first and second filters 18a, 18b are fixed is arranged. Here, filter fixing frame 19 has a rectangular shape as viewed two-dimensionally and is provided in the glucometer main body unit. First and second filters 18a, 18b transmit only light having a wavelength in a particular range and prevent transmittance of light having a wavelength in the other ranges. In the present embodiment, used as first filter 18a is a filter transmitting the mid-infrared radiation with wavelengths of 9 µm-10 µm dependent on a blood glucose concentration, and used as second filter 18b is a filter transmitting the mid-infrared radiation with wavelengths of 8 µm-9 µm independent of a blood glucose concentration. Outlet-side opening portion 14b of waveguide 15 formed in the interior of waveguide formation member 14 faces light-receiving region 17 of light-receiving element 16 with these filters 18a, 18b fixed to this filter fixing frame 19 being interposed.

Filter fixing frame 19 is connected to solenoid 20 and is driven in the direction of arrow A in the figure by solenoid 20. Here, solenoid 20 is an electromagnetic component converting electrical energy into a mechanical linear motion and includes a cylindrically wound coil and a movable iron core put into a hollow portion of the coil. Filter fixing frame 19 is fixed to one end of this movable iron core. It is noted that solenoid 20 is provided in the glucometer main body unit.

As shown in Fig. 2, light-receiving element 16 is connected to a control portion 22 through a light receiving circuit 23 processing an electrical signal output from light-receiving element 16. On the other hand, solenoid 20 is connected to control portion 22 through a drive circuit 24 driving solenoid 20. Control portion 22 outputs a control signal driving and controlling solenoid 20 to drive circuit 24 and receives an input of an amplified electrical signal output from light receiving circuit 23. Then, an operation process is performed based on the amplified electrical signal output from light receiving circuit 23 to calculate a blood glucose concentration.

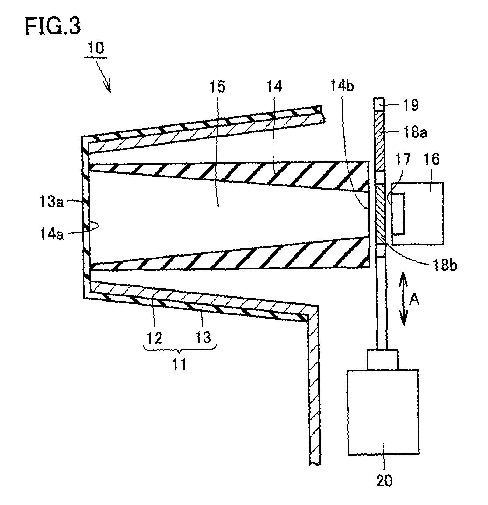

Fig. 3 is a view showing a state after switching in a case where the filters are switched in the biological information measuring sensor shown in Fig. 2. It is noted that in this Fig. 3, the control portion, the light receiving circuit and the drive circuit are not shown. In the following, referring to Fig. 2 and Fig. 3, a filter switching operation will be described.

As shown in Fig. 2, before switching the filters, first filter 18a fixed to the end side of filter fixing frame 19 is positioned between waveguide 15 and light-receiving region 17. This state corresponds to the first state. In this first state, radiation light from eardrum 30, which enters from inlet-side opening portion 14a of waveguide 15, is condensed and exits from outlet-side opening portion 14b of waveguide 15, is applied to filter 18a. Radiation light from eardrum 30 applied to filter 18a is separated by filter 18a, so that only mid-infrared radiation having wavelengths 9 µm-10 µm, which is light having the wavelengths in a first range, is transmitted and this light is then applied to light-receiving region 17. Then, only this light having the wavelengths in the first range is received in light-receiving region 17 and used for spectral detection.

As shown in Fig. 3, after switching the filters, filter fixing frame 19 is driven and moved according to the operation of solenoid 20, so that second filter 18b fixed to the base side of filter fixing frame 19 is positioned between waveguide 15 and light-receiving region 17. This state corresponds to the second state. In this second state, radiation light from eardrum 30, which enters from inlet-side opening portion 14a of waveguide 15, is condensed and exits from outlet-side opening portion 14b of waveguide 15, is applied to filter 18b. Radiation light from eardrum 30 applied to filter 18b is separated by filter 18b, so that only mid-infrared radiation having wavelengths 8 µm-9 µm, which is light having the wavelengths in a second range, is transmitted and this light is then applied to light-receiving region 17. Then, only this light having the wavelengths in the second range is received in light-receiving region 17 and used for spectral detection.

A blood glucose concentration is determined by an operation processing portion provided in control portion 22, based on the spectra detected in these first state and second state. It is noted that filter fixing frame 19 returns to the original point from the state shown in Fig. 3 to the state shown in Fig. 2, after the end of measurement.

Biological information measuring sensor 10 configured as described above uses solenoid 20 to allow filters 18a and 18b to be switched, so that light with wavelengths in a plurality of ranges can be received separately by single light-receiving region 17. Therefore, there is no need for providing a plurality of light-receiving regions 17, and, as a result, the opening diameter of waveguide 15 can be reduced. Therefore, even when light-receiving region 17 is increased in size, the opening diameter of waveguide 15 can be reduced as compared with the case where there are a plurality of light-receiving regions 17, thereby allowing size reduction of probe portion 11. Thus, both improvement of measurement accuracy resulting from enlargement of light-receiving region 17 and size reduction of probe portion 11 can be achieved, resulting in a high-performance biological information measuring sensor. In addition, because of the size reduction of probe portion 11, when probe portion 11 is put into the auditory meatus or the like, the tip end of probe portion 11 can be brought closer to eardrum 30 without coming into contact with the surrounding obstruction, so that inlet-side opening portion 14a of waveguide 15 can be brought into close proximity to eardrum 30. Therefore, more light can be introduced into waveguide 15, and improvement of measurement accuracy is achieved also in this respect.

Furthermore, since the opening area is formed to gradually reduce from inlet-side opening portion 14a toward outlet-side opening portion 14b, radiation light entering waveguide 15 can be condensed in outlet-side opening portion 14b with the required minimum number of times of reflection on the wall surface of waveguide 15. Therefore, absorption or scattering of light involved with reflection is minimized and loss of light is reduced. As a result, higher light-receiving efficiency is realized. Therefore, a blood glucose concentration included in a living tissue can be measured accurately.

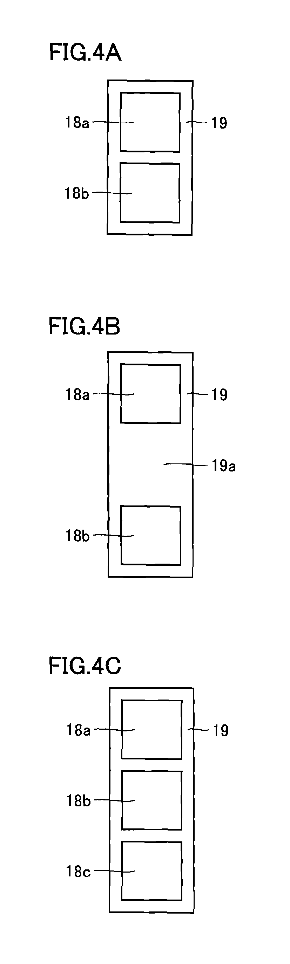

Fig. 4A to Fig. 4C are views showing exemplary layouts of the filters in the present embodiment. Fig. 4A shows a layout of the filters of biological information measuring sensor 10 shown in Fig. 1 to Fig. 3 as described above, in which first and second filters 18a, 18b are arranged adjacent to each other in filter fixing frame 19 having a rectangular shape as viewed two-dimensionally. Here, first and second filters 18a, 18b are arranged side by side in the direction parallel to the driving direction of solenoid 20.

Other than the layout shown in Fig. 4A as described above, the filters can be arranged in a variety of layouts. For example, in a layout shown in Fig. 4B, a shield region 19a is provided between first and second filters 18a and 18b arranged parallel to the driving direction of solenoid 20. Shield region 19a is a part that does not transmit light and is formed, for example, of a part of filter fixing frame 19. In this manner, first and second filters 18a, 18b are laid out in filter fixing frame 19, and solenoid 20 assumes, in addition to the aforementioned first and second states, a third state in which shield region 19a is positioned between waveguide 15 and light-receiving region 17, whereby application of radiation light to light-receiving region 17 is avoided in this third state, thereby enabling monitoring of a room temperature state, detection of a reference output value, and the like. Therefore, such information can be utilized as parameters in measurement of a blood glucose concentration, resulting in a small and high-performance biological information measuring sensor.

Furthermore, as shown in Fig. 4C, three, first, second and third filters 18a, 18b, 18c may be laid out parallel to the driving direction of solenoid 20. In such a layout, not only spectral detection of light having the wavelengths in the first and second ranges but also spectral detection of light having the wavelengths in the third range becomes possible, so that detection of a plurality of components (specifically, a blood glucose concentration and any other component) becomes possible. Here, the number of filters to be arranged may be four or more, as a matter of course, as long as there are more than one.

### (Second Embodiment)

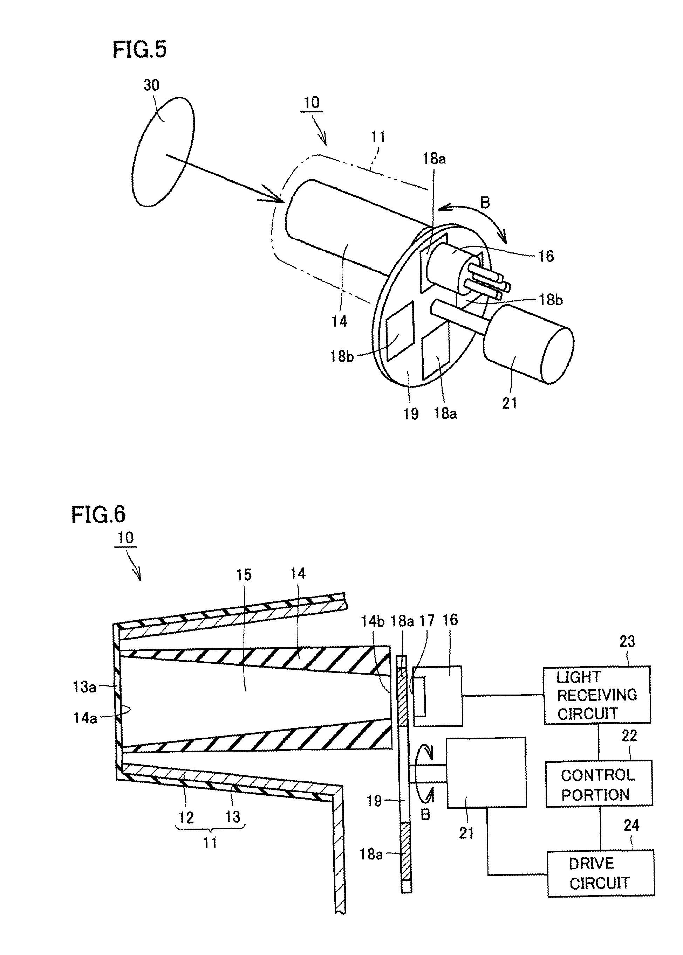

Fig. 5 is a view schematically showing a usage state of a biological information measuring sensor in a second embodiment of the present invention, and Fig. 6 is a schematic cross-sectional view of the biological information measuring sensor shown in Fig. 5. In the following, referring to these figures, the structure of the biological information measuring sensor in the present embodiment will be described. It is noted that the parts similar to those of the biological information measuring sensor in the first embodiment as described above will be denoted with the same reference characters in the figures and the description will not be repeated here.

As shown in Fig. 5 and Fig. 6, in biological information measuring sensor 10 in the present embodiment, solenoid 20 as switching means and drive means in the biological information measuring sensor in the first embodiment as describe above is changed to a motor 21. Then, filter fixing frame 19 to which first and second filters 18a, 18b are fixed is arranged at the back of waveguide formation member 14 and in front of light-receiving region 17. Here, filter fixing frame 19 has a circular shape as viewed two-dimensionally and is provided in the glucometer main body unit.

First and second filters 18a, 18b transmit only light having a wavelength in a particular range and prevent transmittance of light having a wavelength in the other ranges. Also in the present embodiment, similar to the foregoing first embodiment, used as first filter 18a is a filter transmitting the mid-infrared radiation having wavelengths of 9 µm-10 µm dependent on a blood glucose concentration, and used as second filter 18b is a filter transmitting the mid-infrared radiation having wavelengths of 8 µm-9 µm independent of a blood glucose concentration. Outlet-side opening portion 14b of waveguide 15 formed in the interior of waveguide formation member 14 faces light-receiving region 17 of light-receiving element 16 with first or second filter 18a, 18b fixed to this filter fixing frame 19 being interposed.

Filter fixing frame 19 has its central portion connected to a drive shaft of motor 21 and is rotatably driven in the direction of arrow B in the figure by motor 21. Here, motor 21 is an electromagnetic component converting electrical energy into a mechanical rotational motion and includes, for example, a movable iron core having a permanent magnet attached thereto and a stator coil arranged around this movable iron core. Filter fixing frame 19 is fixed to one end of this movable iron core. Here, motor 21 is provided in the glucometer main body unit.

As shown in Fig. 6, light-receiving element 16 is connected to control portion 22 through light receiving circuit 23 processing an electrical signal output from light-receiving element 16. On the other hand, motor 21 is connected to control portion 22 through drive circuit 24 driving motor 21. Control portion 22 outputs a control signal driving and controlling motor 21 to drive circuit 24 and receives an input of an amplified electrical signal output from light receiving circuit 23. Then, an operation process is performed based on the amplified electrical signal output from light receiving circuit 23 to calculate a blood glucose concentration.

By employment of the configuration as described above, filter fixing frame 19 is rotated using motor 21, whereby the filters are switched between the first state in which first filter 18a is arranged between waveguide 15 and light-receiving region 17 and the second state in which second filter 18b is arranged between waveguide 15 and light-receiving region 17, thereby achieving the effect similar to the effect in the foregoing first embodiment.

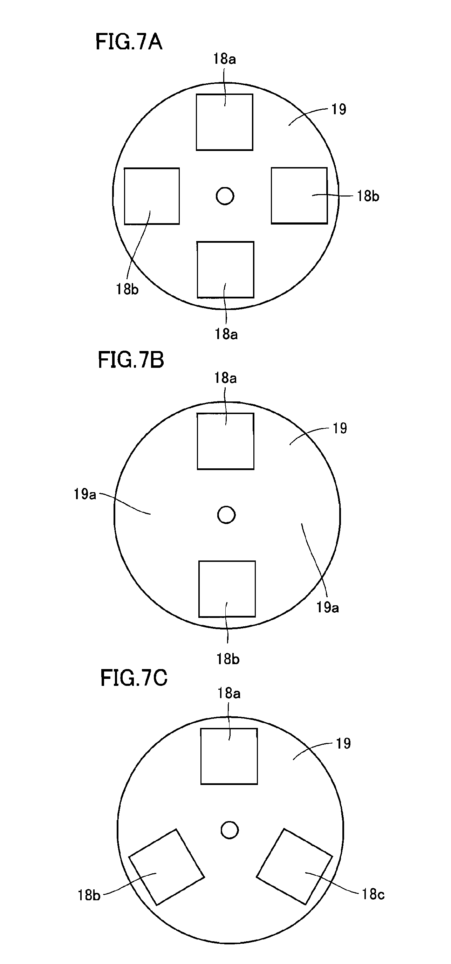

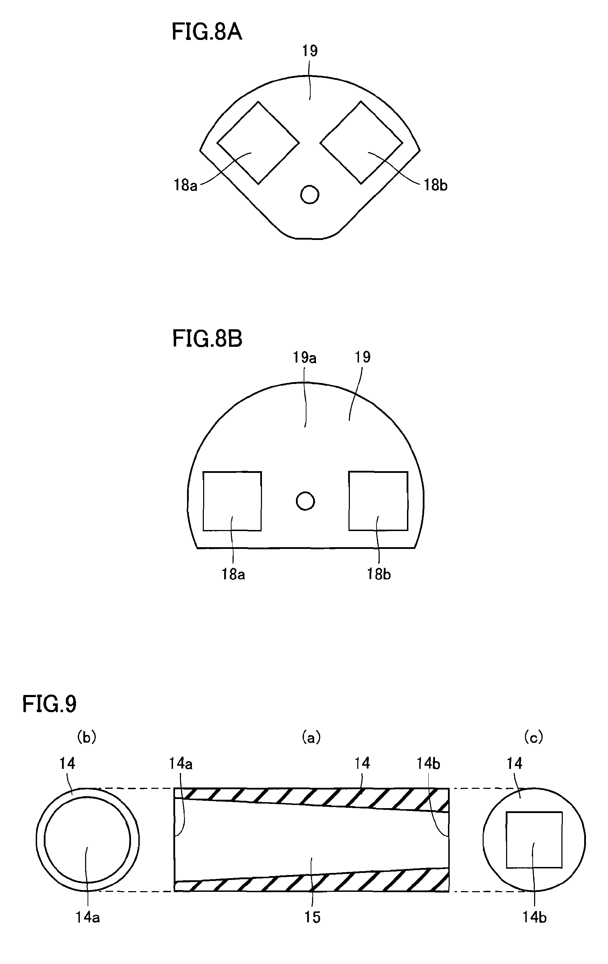

Fig. 7A to Fig. 7C, Fig. 8A and Fig. 8B are views showing exemplary layouts of the filters in the present embodiment. Fig. 7A shows a layout of the filters of biological information measuring sensor 10 shown in Fig. 5 and Fig. 6 as described above, in which first and second filters 18a, 18b are arranged at respective two locations positioned in point symmetry in filter fixing frame 19 having a circular shape as viewed two-dimensionally. It is noted that first and second filters 18a, 18b are arranged side by side in the rotating direction of the rotational shaft of motor 21 (that is, the circumferential direction of filter fixing frame 19).

Other than the layout shown in Fig. 7A as described above, the filters can be arranged in a variety of layouts. For example, in a layout shown in Fig. 7B, only a pair of first and second filters 18a, 18b is arranged in the rotating direction of the rotational shaft of motor 21, and a pair of shield regions 19a is provided between these first and second filters 18a and 18b in the circumferential direction of filter fixing frame 19. Furthermore, as shown in Fig. 7C, three, first, second and third filters 18a, 18b, 18c may be laid out in the rotating direction of the rotational shaft of motor 21. It is noted that the number of filters to be arranged may be four or more, as a matter of course, as long as there are more than one. In addition, as shown in Fig. 8A and Fig. 8B, filter fixing frame 19 may be shaped like a sector as viewed two-dimensionally or may be formed in an approximately semicircular shape.

Fig. 9 is a view showing a modification of the waveguide formation member of the biological information measuring sensor in the first or second embodiment as described above, in which (a) is a side view of the waveguide formation member, (b) is a view showing the shape of the inlet-side end face of the waveguide formation member, and (c) is a view showing the shape of the outlet-side end face of the waveguide formation member. In biological information measuring sensor 10 in the first and second embodiments as described above, the shape of the waveguide may be modified in various manners. For example, the shape of the outlet-side opening portion may be changed according to the shape of the light-receiving region provided in light-receiving element 16. The modification shown in Fig. 9 is an exemplary suitable configuration of waveguide formation member 14 in the case where the light-receiving region of light-receiving element 16 has a rectangular shape.

In the modification shown in Fig. 9, the opening shape of inlet-side opening portion 14a of waveguide formation member 14 and the opening shape of outlet-side opening portion 14b of waveguide formation member 14 are different from each other. More specifically, the opening shape of inlet-side opening portion 14a is circular and the opening shape of outlet-side opening portions 14b is rectangular. In this manner, in the case where inlet-side opening portion 14a and outlet-side opening portion 14b of waveguide 15 have different opening shapes, advantageously, at the inlet side of waveguide 15, the shape of inlet-side opening portion 14a can be selected so that light enters the waveguide to a maximum extent, while at the outlet side of waveguide 15, the shape of outlet-side opening portion 14b can be selected according to the shape of light-receiving region 17 so that the condensed light enters light-receiving region 17 without loss. Therefore, the higher light-receiving efficiency can be realized.

In the foregoing embodiments, the description has been made by way of illustration to the case where the present invention is applied to the biological information measuring sensor incorporated in a glucometer measuring a blood glucose concentration by detecting the spectra of wavelengths in two ranges of mid-infrared radiation. However, the present invention is also applicable to a biological information measuring sensor incorporated in a measuring apparatus detecting any other biological component or a biological information measuring sensor incorporated in a measuring apparatus measuring temperature information, pulse rate, blood pressure, or the like. In the former case, the present invention is additionally applicable to, for example, one using mid-infrared radiation as well as one using near-infrared radiation or one using visible light. Furthermore, the components to be detected include, in addition to glucose included in the blood as described above, hemoglobin, oxyhemoglobin, neutral fat, cholesterol, albumin, uric acid, and the like.

Furthermore, in the foregoing embodiments, the description has been made by way of illustration to the biological information measuring sensor receiving radiation light radiated from a part to be measured of a living body. However, the present invention is also applicable to a biological information measuring sensor receiving transmitted light or reflected light of light applied from a light source to a part to be measured of a living body and photoelectrically converting the same, as a matter of course.

Furthermore, in the foregoing embodiments, the description has been made by way of illustration assuming that a part to be measured of a living body is an eardrum. However, a part to be measured is not limited thereto and may be various parts of a living body.

In addition, in the foregoing embodiments, the description has been made by way of illustration to the case where a filter is employed as spectroscopic means, although a diffraction grating or prism may be used. In the case where a filter is used as spectroscopic means, it is necessary to prepare a plurality of filters and switch them as described above. In the case where a diffraction grating or a prism is used as spectroscopic means, light with wavelengths in different ranges can be separated and derived by preparing a single diffraction grating or prism and rotatably driving them, for example.

In this manner, the foregoing embodiments as disclosed herein are illustrative rather than limitative in all respects. The technical scope of the present invention is defined by the claims, and it is intended that equivalents to the claims and all modifications within the scope are embraced.

## Claims

1. A biological information measuring sensor for measuring biological information in a noninvasive manner by receiving light from a living body, comprising:
a light-receiving region provided in light-receiving means for receiving light from a living body;
a waveguide including an inlet-side opening portion where said light enters and an outlet-side opening portion where said light exits and being provided corresponding to said light-receiving region to introduce said light to said light-receiving region;
spectroscopic means positioned between said light-receiving region and said waveguide to separate said light exiting from said outlet-side opening portion and apply only light having a wavelength in a particular range to said light-receiving region; and
switching means capable of switching said spectroscopic means to at least two or more states including a first state in which only light having a wavelength in a first range of said light is applied to said light-receiving region and a second state in which only light having a wavelength in a second range of said light is applied to said light-receiving region.

2. The biological information measuring sensor according to claim 1, wherein
said spectroscopic means is formed of a plurality of filters at least including a first filter only transmitting light having a wavelength in said first range and a second filter only transmitting light having a wavelength in said second range, and
said switching means switches said plurality of filters such that only a particular filter of said plurality of filters is positioned between said waveguide and said light-receiving means.

3. The biological information measuring sensor according to claim 2, wherein
said plurality of filters are fixed to a filter fixing frame, and
said switching means is formed of drive means for driving said filter fixing frame.

4. The biological information measuring sensor according to claim 3, wherein said filter fixing frame has a shield region which does not transmit light, and
said drive means can drive said filter fixing frame, in addition to said first and second states, to a third state in which said light is not applied to said light-receiving region by positioning said shield region between said waveguide and said light-receiving region.

5. The biological information measuring sensor according to claim 1, wherein said waveguide is formed to have its opening area gradually decreasing from said inlet-side opening portion toward said outlet-side opening portion.

6. The biological information measuring sensor according to claim 1, wherein, in said waveguide, an opening shape in said inlet-side opening portion and an opening shape in said outlet-side opening portion are different from each other.
